# EUROPÄISCHE PATENTANMELDUNG

(11) **EP 2 743 849 A1**
(43) Veröffentlichungstag der Anmeldung: **18.06.2014**
(21) Anmeldenummer: 12196339.1
(22) Anmeldetag: 10.12.2012
(51) Int. Cl.: G06F 19/00

(54) **System zur Förderung der Therapietreue von Patienten**

(71) Anmelder: WEPA Apothekenbedarf GmbH & Co.KG, 56204 Hillscheid (DE)
(72) Erfinder: Heinz Kragt, 56170 Bendorf (DE)
(74) Vertreter: Eisenführ Speiser

(57) **Zusammenfassung**

Die vorliegende Erfindung betrifft ein System zur Förderung der Therapietreue von Patienten. Das System (100) umfasst eine Vorrichtung (10) zum Erstellen eines Therapieplans für einen Patienten durch einen Nutzer, insbesondere durch einen Heilkundigen, wobei der Therapieplan ein geplantes Therapieereignis umfasst. Ferner umfasst das System (100) einen elektronischen Informationsträger (20) für den Patienten, welcher angepasst ist, den Patienten basierend auf dem Therapieplan an das geplante Therapieereignis zu erinnern. Die Vorrichtung (10) ist angepasst, dem Nutzer zu ermöglichen, zu dem geplanten Therapieereignis eine Information zur Beschreibung des geplanten Therapieereignisses in dem Therapieplan bereitzustellen. Der elektronische Informationsträger (20) ist angepasst, die beschreibende Information für den Patienten wiederzugeben, wenn der Patient an das geplante Therapieereignis erinnert wird. Die beschreibende Information kann eine durch den Nutzer frei editierbare Textinformation und/oder eine Audioinformation und/oder eine Standbildinformation und/oder eine Bewegtbildinformation umfassen.

## Beschreibung

Die vorliegende Anmeldung betrifft ein System zur Förderung der Therapietreue von Patienten. Des Weiteren betrifft die vorliegende Anmeldung eine Vorrichtung zum Erstellen eines Therapieplans für einen Patienten durch einen Nutzer, insbesondere durch einen Heilkundigen, zur Verwendung in dem System zur Förderung der Therapietreue von Patienten und einen elektronischen Informationsträger für einen Patienten zur Verwendung in dem System zur Förderung der Therapietreue von Patienten.

Entsprechend einer aktuellen Schätzung der Bundesvereinigung Deutscher Apothekerverbände (ABDA) wird etwa ein Viertel aller verschriebenen Medikamente nicht oder nicht wie verordnet eingenommen. Man spricht in diesem Zusammenhang auch von einer mangelnden Compliance bzw. Komplianz (wörtlich etwa als "Folgsamkeit" oder "Fügsamkeit" zu übersetzen) des Patienten als Oberbegriff für dessen kooperatives Verhalten in einer Therapie. In der vorliegenden Anmeldung wird hierfür der ebenfalls geläufige und deutlich griffigere Begriff der "Therapietreue" verwendet. (Eine weitere, im Englischen oft synonym verwendete Bezeichnung ist Adherence bzw. Adhärenz; in der Literatur findet sich zudem auch der Begriff Konkordanz.) Eine gute Therapietreue entspricht einem konsequenten Befolgen der ärztlichen Ratschläge; laut der Weltgesundheitsorganisation (engl. "World Heath Organisation", WHO) erreichen diese im Durchschnitt aber nur etwa 50% der Patienten.

Die Gründe für eine mangelnde Therapietreue (Non-Compliance) sind vielschichtig. Hier spielen zum Teil psychologische Faktoren, wie etwa ein mangelndes Vertrauensverhältnis zwischen dem Patienten und dem behandelnden Arzt bzw. Apotheker, ein fehlender, unmittelbarer Leidensdruck bei Patienten, die sich eigentlich gesund fühlen, Mechanismen der Krankheitsverdrängung, usw., eine wichtige Rolle. Gerade älteren, insbesondere auch mehrfach und/oder chronisch erkrankten Patienten (man spricht hier auch von Multimorbidität), die möglicherweise mehrmals täglich verschiedene Medikamente in ggf. unterschiedlichen Dosierungen einnehmen oder anwenden müssen, fällt es oft aber auch einfach schwer, die vorgegebene Medikation wie verschrieben, und somit wirksam und zielführend, umzusetzen. Es kann dann etwa vorkommen, dass ein Medikament zum falschen Zeitpunkt oder - zumeist noch schlimmer - auch gar nicht eingenommen bzw. angewendet wird. Zudem kann es zu Verwechslungen kommen, wenn sich die Patienten z.B. die Tablettenformen, die Produktnamen, usw. nicht richtig merken bzw. diese nicht richtig auseinanderhalten können. Hiervon sind insbesondere Patienten betroffen, die alleine leben und nur teilweise, in der Regel am Tag, betreut werden (können).

Durch die beschriebenen Probleme in Bezug auf die Therapietreue entstehen Gefahren für das Wohl der Patienten. In Abhängigkeit von der Grunderkrankung kann z.B. eine fehlerhafte Medikamenteneinnahme mit einem erhöhten Sterberisiko, mit mehr und/oder stärkeren Krankheitssymptomen und/oder mit einer geringeren Lebensqualität einhergehen. Als ein weiterer negativer Aspekt sind die hierdurch entstehenden zusätzlichen Folge- bzw. Zusatzkosten für die medizinischen Leistungsträger (z.B. Krankenkassen) zu nennen, die beispielsweise in Deutschland bundesweit auf zwei- bis dreistellige Millionenbeträge pro Jahr geschätzt werden.

Zur Förderung der Therapietreue wird den Patienten häufig (aber nicht zwangsweise) ein sogenannter Medikamentenplan ausgehändigt, auf dem mehr oder weniger übersichtlich die oral einzunehmenden Medikamente sowie deren Dosierungen und Einnahmezeiten, ggf. mit weiteren Hinweisen zur Einnahme, wie etwa "vor der Mahlzeit", "zur Mahlzeit" oder "nach der Mahlzeit", schriftlich aufgeführt sind. Hierbei kann es jedoch zu Problemen kommen, wenn die Einnahmehinweise zu "wissenschaftlich" und insbesondere unter Verwendung von Fachausdrücken, wie etwa "orale Applikation" oder dergleichen, formuliert sind, da diese den Patienten häufig nicht geläufig bzw. in ihrer Bedeutung nicht im Detail verständlich sind. Des Weiteren werden in herkömmlichen Medikamentenplänen weitere Anwendungen von nicht-oralen Medikamenten, wie etwa das Auftragen einer Salbe auf die Haut, das Einträufeln von Tropfen in das/die Auge(n), usw., in der Regel kaum bzw. gar nicht erfasst. Außerdem liefert ein Medikamentenplan einem Patienten zwar die Information welche Medikamente er wann wie einnehmen soll; er unterstützt den Patienten aber praktisch gar nicht dabei, zu den vorgesehenen Einnahmezeiten auch wirklich an die Einnahme der Medikamente zu denken.

Neben den genannten Medikamentenplänen sind daher heute bereits verschiedene elektronische Geräte bzw. Systeme verfügbar, die Patienten bei der korrekten Einnahme von Medikamenten unterstützen sollen. Beispielsweise ist von der Firma FONIUM Deutschland GmbH ein tragbares Gerät (derBULTER®) erhältlich, das eine Funktion als Medikamenten-Timer aufweist und das Patienten über eine Meldung an die Medikamenteneinnahme erinnert. Des Weiteren ist vom Wort & Bild Verlag, dem Herausgeber der Apotheken UMSCHAU unter der Bezeichnung "Apotheke vor Ort" eine App erhältlich, die es Patienten unter anderem ermöglicht, auf einem entsprechenden Smartphone, z.B. einem Apple® iPhone®, eine persönliche Medikamentenliste zu erstellen.

Die genannten Systeme bzw. Geräte haben jedoch zum Teil den Nachteil, dass sie in der Anwendung sehr komplex sind. Beispielsweise wird vom Nutzer der gelernte Umgang mit modernsten Kommunikationsmitteln, wie etwa PDAs ("Personal Digital Assistent") oder Smartphones, vorausgesetzt, was gerade für ältere Patienten auch heutzutage noch längst keine Selbstverständlichkeit ist. Des Weiteren müssen die Medikamentendaten vom Nutzer teilweise selbst angelegt bzw. verwaltet werden; dies kann ebenfalls nicht von allen Patienten geleistet werden. In einigen Fällen ist darüberhinaus vorgesehen, dass Aktualisierungen von Daten per Datenfernübertragung, z.B. über eine Internetverbindung, vorgenommen werden, was nicht immer technisch durchführbar ist.

Es ist daher eine Aufgabe der vorliegenden Erfindung, ein System zur Förderung der Therapietreue von Patienten vorzusehen, das geeignet ist, das kooperative Verhalten gerade älterer, insbesondere auch mehrfach und/oder chronisch erkrankter Patienten in einer Therapie weiter zu verbessern. Des Weiteren ist es eine Aufgabe der vorliegenden Erfindung eine Vorrichtung zum Erstellen eines Therapieplans für einen Patienten, insbesondere durch einen Heilkundigen, zur Verwendung in dem System zur Förderung der Therapietreue von Patienten und einen elektronischen Informationsträger für einen Patienten zur Verwendung in dem System zur Förderung der Therapietreue von Patienten vorzusehen.

Gemäß einem ersten Aspekt der vorliegenden Erfindung wird ein System zur Förderung der Therapietreue von Patienten bereitgestellt, wobei das System umfasst:
- eine Vorrichtung zum Erstellen eines Therapieplans für einen Patienten durch einen Nutzer, insbesondere durch einen Heilkundigen, wobei der Therapieplan ein geplantes Therapieereignis umfasst, und
- einen elektronischen Informationsträger für den Patienten, welcher angepasst ist, den Patienten basierend auf dem Therapieplan an das geplante Therapieereignis zu erinnern,
wobei die Vorrichtung angepasst ist, dem Nutzer zu ermöglichen, zu dem geplanten Therapieereignis eine Information zur Beschreibung des geplanten Therapieereignisses in dem Therapieplan bereitzustellen,
wobei der elektronische Informationsträger angepasst ist, die beschreibende Information für den Patienten wiederzugeben, wenn der Patient an das geplante Therapieereignis erinnert wird, und
wobei die beschreibende Information eine durch den Nutzer frei editierbare Textinformation und/oder eine Audioinformation und/oder eine Standbildinformation und/oder eine Bewegtbildinformation umfassen kann.

Unter dem Begriff "Heilkundiger" werden in der vorliegenden Anmeldung insbesondere Ärzte und Apotheker, ganz allgemein aber auch andere im Bereich der Heilkunde tätige Personen, wie etwa Zahnärzte, Tierärzte, Psychotherapeuten, Physiotherapeuten, Ergotherapeuten, Osteopathen, Heilpraktiker, Logopäden, Motopäden usw. verstanden. Die Vorrichtung zum Erstellen eines Therapieplans für einen Patienten durch einen Nutzer kann also beispielsweise von einem Arzt in einer Praxis oder in einem Krankenhaus, von einem Apotheker in einer Apotheke oder von einem Physiotherapeuten in einer physiotherapeutischen Einrichtung verwendet werden.

Der Begriff "Therapieereignis" umfasst hier beispielsweise die orale Einnahme eines Medikamentes und/oder die Anwendung eines nicht-oralen Medikamentes, wie etwa das Auftragen einer Salbe auf die Haut, das Einträufeln von Tropfen in das/die Auge(n), usw., z.B. jeweils zu einer festgelegten Uhrzeit und ggf. mehrfach innerhalb eines festgelegten einmaligen oder sich wiederholenden Zeitraums, wie etwa zweimal die Woche, immer Montag und Donnerstag. Zusätzlich oder alternativ kann es sich bei einem Therapieereignis aber z.B. auch um einen, möglicherweise regelmäßigen, Termin zu einer Behandlung bei einem Heilkundigen, wie etwa einem Arzt oder einem Physiotherapeuten, oder aber auch nur um eine der Therapie förderliche, ggf. regelmäßig auszuführende, kurze Folge von Übungen, wie etwa Entspannungsübungen, oder um ein ähnliches Ereignis handeln.

Die Vorrichtung zum Erstellen eines Therapieplans für einen Patienten durch einen Nutzer kann ein Computer, z.B. ein Festplatzcomputer, ein Notebook oder ein Tablet-Computer, sein, auf dem Programmcodemittel eines Computerprogramms ausgeführt werden, welche den Computer zu einer Vorrichtung zum Erstellen eines Therapieplans für einen Patienten durch einen Nutzer, insbesondere durch einen Heilkundigen, zur Verwendung in dem System zur Förderung der Therapietreue von Patienten weiterbilden.

Der elektronische Informationsträger für den Patienten kann ein elektronisches Gerät sein, dessen Abmessungen bzw. dessen Gestalt im Wesentlichen denjenigen heutiger Smartphones entsprechen. Das Gerät kann unter anderem ein Display zur Wiedergabe visueller Informationen und/oder einen Lautsprecher zur Wiedergabe akustischer Informationen umfassen. Eine geeignete Displaygröße beträgt z.B. um die 5 Zoll (zirka 12,7 cm) Displaydiagonale. Das Display kann vorzugsweise als Touch-Screen-Display ausgebildet sein, um auf einfache Weise Eingaben durch den Patienten zu ermöglichen. Die Funktionalität des Erinnerns des Patienten an das geplante Therapieereignis kann beispielsweise durch einen visuellen und/oder einen akustischen Alarm (z.B. ein Blinken und/oder ein Piepen), welcher zu einem in dem Therapieplan für das Therapieereignis vorgesehenen Zeitpunkt ausgelöst wird, realisiert sein.

Es kann vorgesehen sein, dass der durch den Nutzer für den Patienten erstellte Therapieplan mittels einer direkten drahtgebundenen Verbindung, z.B. einem USB- oder Firewire-Kabel oder dergleichen, auf den elektronischen Informationsträger für den Patienten übertragen und dort gespeichert wird. Dies hätte den Vorteil, dass das Übertragen eines jeweils neuen Therapieplans immer auch einen Kontakt zwischen dem Nutzer, z.B. einem Arzt oder Apotheker, erforderlich macht, was helfen kann, die Betreuung des Patienten durch den Nutzer zu verbessern. Alternativ oder zusätzlich kann aber auch eine "indirekte" Übertragung des Therapieplans mittels eines portablen Speichermediums, wie etwa einem USB-Speicherstick, einer USB-Festplatte oder dergleichen, möglich sein. In diesem Fall könnte der Nutzer den Therapieplan zunächst auf das portable Speichermedium kopieren und der Nutzer (oder eine andere geeignete Person, wie etwa eine Krankenschwester oder eine Mitarbeiterin eines ambulanten Pflegedienstes) könnte den Therapieplan dann bei dem Patienten vor Ort, z.B. in einem Krankenhaus oder Pflegeheim, auf den elektronischen Informationsträger für den Patienten übertragen und dort speichern. Auch in diesem Fall gäbe es also einen persönlichen Kontakt mit dem Patienten. Grundsätzlich wäre es zusätzlich oder alternativ aber natürlich auch möglich, dass der Therapieplan mittels einer indirekten und/oder drahtlosen Verbindung, z.B. einer Datenfernübertragung, etwa über einem Mobilfunkdienst, auf den elektronischen Informationsträger für den Patienten übertragen wird. Dies würde aber ggf. den Abschluss eines Mobilfunkvertrages bzw. einen drahtlosen oder drahtgebunden Internetanschluss erfordern, was zum einen mit zusätzlichen Kosten für den Patienten verbunden ist und zum anderen auch keine so unmittelbare Betreuung des Patienten durch den Nutzer garantiert.

Die Erfindung betrifft den Gedanken, in einem System zur Förderung der Therapietreue von Patienten eine Vorrichtung zum Erstellen eines Therapieplans für einen Patienten durch einen Nutzer, insbesondere einen Heilkundigen, vorzusehen, die es dem Nutzer, z.B. einem Arzt oder Apotheker, ermöglicht, zu einem von dem Therapieplan umfassten geplanten Therapieereignis eine Information zur Beschreibung des geplanten Therapieereignisses in dem Therapieplan bereitzustellen, wobei die beschreibende Information eine durch den Nutzer frei editierbare Textinformation und/oder eine Audioinformation und/oder eine Standbildinformation und/oder eine Bewegtbildinformation umfassen kann. Die beschreibende Information wird von einem ebenfalls in dem System zur Förderung der Therapietreue von Patienten für den Patienten vorgesehenen elektronischen Informationsträger für den Patienten wiedergegeben, wenn der Patient basierend auf dem Therapieplan durch den elektronischen Informationsträger an das geplante Therapieereignis erinnert wird. Beispielsweise kann, wenn das geplante Therapieereignis etwa die orale Einnahme eines Medikamentes ist, das in Tablettenform dargereicht wird, der Nutzer als die beschreibende Information, oder als ein Teil derselben, eine durch den Nutzer frei editierbare Textinformation, wie etwa den Satz "Einnahme mit einem Glas Wasser", und/oder eine Standbildinformation, welche z.B. die Verpackung des einzunehmenden Medikamentes und/oder einen Blister und/oder eine entsprechende Tablette zeigt, in dem Therapieplan bereitstellen. Durch Letzteres kann die Wahrscheinlichkeit reduziert werden, dass der Patient Medikamente verwechselt, wenn er sich z.B. die Tablettenformen, die Produktnamen, usw. nicht richtig merken bzw. diese nicht richtig auseinanderhalten kann. Demgegenüber kann die Bereitstellung einer Audioinformation und/oder einer Bewegtbildinformation als die beschreibende Information zu einem von dem Therapieplan umfassten geplanten Therapieereignis insbesondere dann vorteilhaft sein, wenn mit der Durchführung des geplanten Therapieereignisses für den Patienten Schwierigkeiten verbunden sind und/oder die Durchführung für ihn ungewohnt ist. Ein gutes Beispiel ist etwa die Injektion eines nicht-oralen Medikamentes mit Hilfe einer Spritze. Hier könnte der Nutzer mittels einer Audioinformation und/oder einer Bewegtbildinformation z.B. die Handhabung der Spritze, mögliche Einstichstellen oder auch den Vorgang der Injektion selbst für den Patienten illustrieren. Als ein anderes Beispiel könnte man sich die Durchführung therapeutischer Übungen, wie etwa Entspannungsübungen und/oder Übungen zur Stärkung bestimmter Muskeln, vorstellen, die mit Hilfe einer Audioinformation und/oder einer Bewegtbildinformation für den Patienten veranschaulicht werden. Insgesamt kann durch die Bereitstellung einer solchen beschreibenden Information, die den Patienten bei der Durchführung eines geplanten Therapieereignisses unterstützt, das kooperative Verhalten gerade älterer, insbesondere auch mehrfach und/oder chronisch erkrankter Patienten in einer Therapie weiter verbessert werden.

Es ist bevorzugt, dass die Vorrichtung eine Kamera zur Aufnahme der Standbildinformation umfasst und angepasst ist, eine Bewegtbildinformation von der Kamera als Vorschau für den Nutzer zu erhalten und wiederzugeben, und dem Nutzer zu ermöglichen, die Standbildinformation aus der als Vorschau erhaltenen und wiedergegebenen Bewegtbildinformation zu erfassen. Wenn die Vorrichtung eine solche Kamera umfasst, kann der Nutzer die Standbildinformation selbst erstellen. Dies ist z.B. vorteilhaft, wenn sich die Verpackung eines Medikamentes ändert und ansonsten noch keine aktuellen Bilder der geänderten Verpackung (kommerziell) verfügbar sind, oder wenn der Nutzer, z.B. ein Arzt oder Apotheker, eine Arznei für den Patienten selbst zubereitet. Zudem ist es durch die Wiedergabe einer Bewegtbildinformation von der Kamera als Vorschau für den Nutzer, aus welcher der Nutzer die Standbildinformation erfassen kann, für den Nutzer sehr einfach, eine geeignete Standbildinformation zu erfassen, die z.B. die Verpackung eines Medikamentes und/oder einen Blister und/oder eine entsprechende Tablette in einer von dem Nutzer gewünschten Anordnung oder Perspektive zeigt.

Es ist des Weiteren bevorzugt, dass die Vorrichtung angepasst ist, einen in der Standbildinformation und/oder in der Bewegtbildinformation sichtbaren Code, insbesondere einen Strichcode und/oder einen Matrixcode, zu erkennen und zu interpretieren, und dem Nutzer zu ermöglichen, die beschreibende Information basierend auf dem erkannten und interpretierten Code bereitzustellen. Der Code kann beispielsweise die auf der Verpackung eines Medikamentes aufgedruckte Klartext-Darstellung (d.h., eine Zahlenfolge mit einem vorangestelltem Minuszeichen und der vorangestellten Buchstabenfolge "PZN") der Pharmazentralnummer (PZN) des Medikamentes oder die entsprechende Darstellung als Strichcode sein. Die PZN ist ein Identifikationsschlüssel, der in Deutschland verwendet wird, um Medikamente bundeseinheitlich nach Bezeichnung, Darreichungsform, Wirkstoffstärke und Verpackungsgröße eindeutig zu kennzeichnen. Die in der Pharmazentralnummer codierten Informationen können von der Vorrichtung beispielsweise dazu verwendet werden, dem Nutzer Vorschläge für die beschreibende Information zu unterbreiten. Dies kann z.B. dadurch geschehen, dass aus einer, z.B. in Form einer Datenbank, angelegten Sammlung von schon einmal in einem Therapieplan bereitgestellten beschreibenden Informationen (siehe unten), solche beschreibenden Informationen, die sich auf die Einnahme oder Anwendung des durch die erkannte und interpretierte PZN gekennzeichneten Medikamentes beziehen, ausgewählt und für den Nutzer in Form eines Auswahlmenüs, über das er die von ihm gewünschte beschreibende Information übernehmen kann, bereitgestellt werden.

Es ist bevorzugt, dass die Vorrichtung angepasst ist, eine Vorschau der Wiedergabe der beschreibenden Information, wie sie durch den elektronischen Informationsträger für den Patienten erfolgen soll, für den Nutzer wiederzugeben. Der Nutzer kann so direkt sehen, wie die beschreibende Information später für den Patienten wiedergeben wird. Dies hilft dem Nutzer, die Information zur Beschreibung des geplanten Therapieereignisses so in dem Therapieplan bereitzustellen, dass deren visuelle und/oder akustische Wiedergabe für den Patienten diesem auch tatsächlich eine Hilfe ist (und nicht z.B. zu klein, und damit nicht lesbar, oder von einer zu schlechten Bild- und/oder Tonqualität ist).

Es ist ferner bevorzugt, dass die Vorrichtung angepasst ist, die durch den Nutzer in dem Therapieplan bereitgestellte beschreibende Information zur möglichen Wiederverwendung automatisch abzuspeichern. Auf diese Weise kann im Laufe der Zeit automatisch eine Sammlung von schon einmal in einem Therapieplan bereitgestellten beschreibenden Informationen, z.B. in Form einer Datenbank, angelegt werden, auf die der Nutzer bei der Erstellung eines Therapieplans für einen Patienten zurückgreifen kann. Dies vereinfacht und beschleunigt das Erstellen von Therapieplänen durch den Nutzer.

Es ist bevorzugt, dass der elektronische Informationsträger angepasst ist, eine Textinformation der beschreibenden Information mittels eines Text-nach-Sprache-Wandlers akustisch wiederzugeben. Auf diese Weise kann beispielsweise eine durch den Nutzer frei editierbare Textinformation, wie etwa der Satz "Einnahme mit einem Glas Wasser", die durch den Nutzer als die beschreibende Information, oder als ein Teil derselben, in dem Therapieplan bereitgestellt wird, für den Patienten vorgelesen werden. Dies ist insbesondere bei Patienten von Vorteil, deren Sehvermögen - sei es aufgrund ihres Alters oder aus einem sonstigen Grund - beeinträchtigt ist.

Es ist ferner bevorzugt, dass der elektronische Informationsträger angepasst ist, von dem Patienten eine Bestätigung zu fordern, dass der Patient an das geplante Therapieereignis erinnert worden ist, und
- einen Bestätigungsnachweis für die durch den Patienten erfolgte Bestätigung zu speichern und/oder per Datenfernübertragung zu der Vorrichtung zu übertragen, und/oder
- die Audioinformation und/oder die Bewegtbildinformation solange wiederholt wiederzugeben, bis die Bestätigung durch den Patienten erfolgt ist.

Die Bestätigungsfunktionalität kann beispielsweise dadurch realisiert sein, dass der Patient als Nutzerschnittstelle einen Bestätigungs-Button (der in dem Fall, dass der elektronische Informationsträger ein Touch-Screen-Display umfasst, z.B. am unteren Rand des Displays als grafisches Element vorgesehen sein kann) drücken muss, um den Alarm auszustellen. (Ein als grafisches Element vorgesehener Bestätigungs-Button kann vorzugsweise zusammen mit - z.B. unterhalb - der beschreibenden Information für den Patienten wiedergegeben werden, wenn der Patient an das geplante Therapieereignis erinnert wird.)

Es ist bevorzugt, dass das Therapieereignis die Einnahme oder Anwendung eines Medikamentes umfasst, wobei der elektronische Informationsträger angepasst ist, basierend auf dem Bestätigungsnachweis eine Prognose über den dem Patienten verbleibenden Vorrat des Medikamentes aufzustellen, wobei der elektronische Informationsträger des Weiteren angepasst ist, die Prognose visuell und/oder akustisch wiederzugeben und/oder per Datenfernübertragung zu der Vorrichtung zu übertragen. Auf diese Weise kann der Patient und/oder der Nutzer der Vorrichtung auf einfache Weise über den jeweils aktuellen Medikamentenvorrat des Patienten auf dem Laufenden gehalten werden. Dies hilft zum einen dem Patienten, da es ihn dabei unterstützt, sich rechtzeitig um ein neues Medikament zu kümmern, zum anderen kann es dem Nutzer der Vorrichtung, z.B. einem Arzt oder Apotheker, helfen, die Lagersteuerung zu verbessern.

Es ist ferner bevorzugt, dass der Therapieplan ein oder mehrere geplante Therapieereignisse umfasst, wobei der elektronische Informationsträger angepasst ist, eine Gesamtübersicht über die ein oder mehreren geplanten Therapieereignisse visuell und/oder akustisch wiederzugeben. Dies ermöglicht einen einfachen und schnellen Überblick über das/die geplante(n) Therapieereignis(se), beispielsweise, wenn der Patient im Urlaub oder auf einer anderen Reise in einer ihm fremden Apotheke ein von ihm benötigtes Medikament besorgen möchte. In einer möglichen Realisierung weist der elektronische Informationsträger als Nutzerschnittstelle einen Informations-Button auf (der in dem Fall, dass der elektronische Informationsträger ein Touch-Screen-Display umfasst, z.B. am unteren rechten Rand des Displays als grafisches Element vorgesehen sein kann). Wird dieser Informations-Button gedrückt, wird die Gesamtübersicht über die ein oder mehreren geplanten Therapieereignisse z.B. als Übersichtsliste oder in der Art einer "Slide-Show" (d.h., ein geplantes Therapieereignis zeitlich nach dem anderen) auf dem Display wiedergegeben. Zusätzlich kann vorgesehen sein, dass eine Information über die "Heimat"-Apotheke des Patienten, z.B. die komplette Adresse der Apotheke, auf dem Display wiedergegeben wird.

Es ist bevorzugt, dass das System angepasst ist
- eine unbefugte Wiederverwendung zumindest eines Teils der in dem Therapieplan bereitgestellten beschreibenden Information zu verhindern, und/oder
- die Wiederwendung der in dem Therapieplan bereitgestellten beschreibenden Information nach einer Wiedergabe einer Erklärung über mögliche bestehende Rechte an zumindest einem Teil der in dem Therapieplan bereitgestellten beschreibenden Information zu ermöglichen.

Zum Beispiel können von dem Nutzer, z.B. einem Arzt oder Apotheker, frei editierte Texte und/oder selbst erstellte Audioinformationen und/oder selbst erstellte Standbildinformationen und/oder selbst erstellte Bewegtbildinformationen verschlüsselt sein, wobei ein anderer Nutzer, z.B. ein anderer Arzt oder Apotheker, der eine der Vorrichtung 10 entsprechende Vorrichtung nutzt, zwar den Therapieplan von dem elektronischen Informationsträger 10 einlesen und ggf. für den Patienten anpassen kann, er aber eben nicht die von dem ersten Nutzer selbst erstellte(n) beschreibende(n) Information(en) wiederverwenden kann. Zusätzlich oder alternativ ist es aber auch möglich, dass eine Erklärung über mögliche bestehende Rechte (hier z.B. des ersten Nutzers) an zumindest einem Teil der in dem Therapieplan bereitgestellten beschreibenden Information (hier der von dem ersten Nutzer selbst erstellten beschreibenden Information) wiedergegeben wird und danach die Wiederverwendung (der gesamten) in dem Therapieplan bereitgestellten beschreibenden Information ermöglicht wird. Die genannte Erklärung kann auch einen Hinweis auf den möglichen Rechteinhaber, hier den ersten Nutzer, z.B. auf seine Praxis oder sein Apotheke, beinhalten, so dass der zweite Nutzer, der die in dem Therapieplan von dem ersten Nutzer bereitgestellte beschreibende Information wiederverwenden möchte, diesen kontaktieren kann, um ggf. die Erlaubnis des Rechteinhabers einzuholen.

Es ist ferner bevorzugt, dass der elektronische Informationsträger ein tragbares elektronisches Gerät ist, bei dem die Funktionalität des Erinnerns des Patienten an das geplante Therapieereignis und der Wiedergabe der beschreibenden Information für den Patienten gegenüber möglichen weiteren Funktionen im Vordergrund steht. Das heißt, der elektronische Informationsträger ist bevorzugt eben nicht einfach ein Tablet-PC, ein PDA oder ein Smartphone, bei dem die entsprechende Funktionalität z.B. in Form einer von möglicherweise vielen weiteren Apps bereitgestellt ist, wobei der Patient die entsprechende App bei jedem Systemstart zunächst erst auswählen und starten muss. Zwar kann, in einer möglichen Realisierung, auch z.B. ein Tablet-PC mit einem entsprechenden Computerprogramm (eben z.B. einer App) als der elektronische Informationsträger verwendet werden; das Computerprogramm läuft dann aber bevorzugt direkt nach dem Start des Betriebssystems des Tablet-PCs automatisch hoch. Der Patient muss in dieser Realisierung also im Wesentlichen nichts anderes machen, als den Tablet-PC einzuschalten; der elektronische Informationsträger ist dann sofort betriebsbereit. Es ist dabei ferner bevorzugt, dass es keine Möglichkeit gibt, das Computerprogramm z.B. aus Versehen vorzeitig zu beenden. Außerhalb der Zeitpunkte, zu denen der elektronische Informationsträger den Patienten an das geplante Therapieereignis erinnert, kann beispielsweise eine Uhr mit Datum auf dem Display des Tablet-PCs wiedergegeben werden. Das System bleibt also jeweils bis zu einer jeweiligen Ausführung der Erinnerungsfunktionalität im Hintergrund und "verschwindet" danach automatisch wieder.

Gemäß einem weiteren Aspekt der vorliegenden Erfindung wird eine Vorrichtung zum Erstellen eines Therapieplans für einen Patienten durch einen Nutzer, insbesondere durch einen Heilkundigen, zur Verwendung in dem System zur Förderung der Therapietreue von Patienten gemäß einem der Ansprüche 1 bis 11 bereitgestellt.

Gemäß noch einem weiteren Aspekt der vorliegenden Erfindung wird ein elektronischer Informationsträger für einen Patienten zur Verwendung in dem System zur Förderung der Therapietreue von Patienten gemäß einem der Ansprüche 1 bis 11 bereitgestellt.

Gemäß noch einem weiteren Aspekt der vorliegenden Erfindung wird ein Computerprogramm vorgesehen, wobei das Computerprogramm Programmcodemittel umfasst, welche, wenn sie auf einem Computer ausgeführt werden, den Computer zu einer Vorrichtung zum Erstellen eines Therapieplans für einen Patienten durch einen Nutzer, insbesondere durch einen Heilkundigen, zur Verwendung in dem System zur Förderung der Therapietreue von Patienten gemäß einem der Ansprüche 1 bis 11 weiterbilden.

Gemäß noch einem weiteren Aspekt der vorliegenden Erfindung wird ein Computerprogramm vorgesehen, wobei das Computerprogramm Programmcodemittel umfasst, welche, wenn sie auf einem tragbaren elektronischen Gerät mit Computerfunktionalität ausgeführt werden, das tragbare elektronische Gerät mit Computerfunktionalität zu einem elektronischen Informationsträger für einen Patienten zur Verwendung in dem System zur Förderung der Therapietreue von Patienten gemäß einem der Ansprüche 1 bis 11 weiterbilden.

Unter dem Begriff "tragbares elektronisches Gerät mit Computerfunktionalität" wird hier ein Tablet-PC, ein PDA, ein Smartphone oder ein ähnliches, mobil verwendbares Gerät verstanden.

Es versteht sich, dass das System nach Anspruch 1, die Vorrichtung nach Anspruch 12, der elektronische Informationsträger nach Anspruch 13, das Computerprogramm nach Anspruch 14 und das Computerprogramm nach Anspruch 15 ähnliche und/oder identische bevorzugte Ausführungsformen, insbesondere wie in den abhängigen Ansprüchen definiert, haben.

Es versteht sich, dass eine bevorzugte Ausführungsform der Erfindung auch jede Kombination der abhängigen Ansprüche mit dem entsprechenden unabhängigen Anspruch sein kann.

Diese und andere Aspekte der Erfindung werden mit Bezug auf die nachfolgend beschriebenen Figuren erläutert, wobei
- Fig. 1: schematisch und exemplarisch eine Ausführungsform eines Systems zur Förderung der Therapietreue von Patienten zeigt, und
- Fig. 2: schematisch und exemplarisch eine Detailansicht des elektronischen Informationsträgers der in der Fig. 1 gezeigten Ausführungsform eines Systems zur Förderung der Therapietreue von Patienten zeigt.

In den Figuren sind gleiche bzw. sich entsprechende Elemente oder Einheiten jeweils mit gleichen bzw. sich entsprechenden Bezugszeichen versehen. Wenn ein Element oder eine Einheit bereits im Zusammenhang mit einer Figur beschrieben worden ist, wird ggf. im Zusammenhang mit einer anderen Figur auf eine ausführliche Darstellung verzichtet.

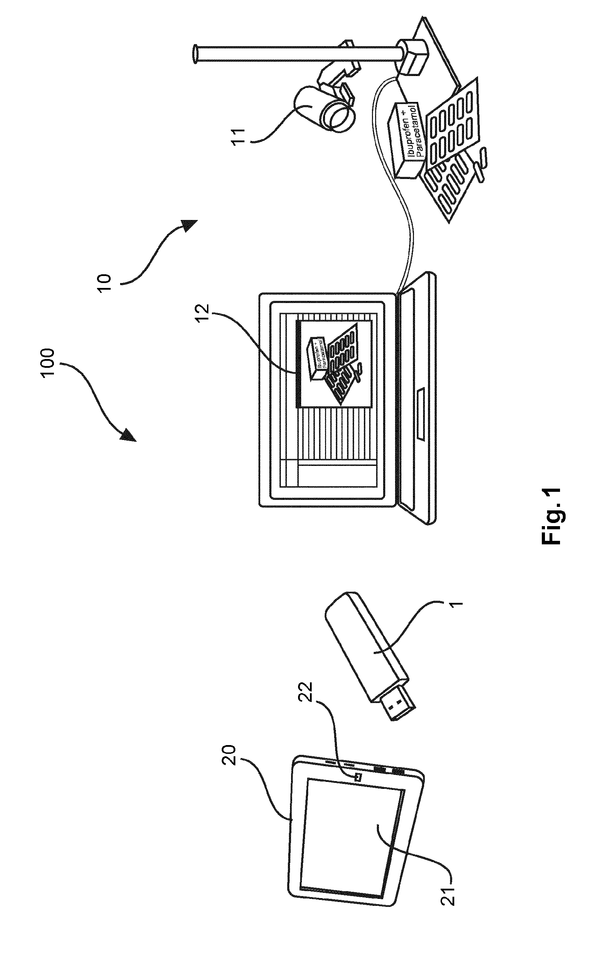

Fig. 1 zeigt schematisch und exemplarisch eine Ausführungsform eines Systems 100 zur Förderung der Therapietreue von Patienten. Das System 100 umfasst hier eine Vorrichtung 10 zum Erstellen eines Therapieplans für einen Patienten durch einen Nutzer, insbesondere durch einen Heilkundigen, wobei der Therapieplan ein geplantes Therapieereignis umfasst, und einen elektronischen Informationsträger 20 für den Patienten, welcher angepasst ist, den Patienten basierend auf dem Therapieplan an das geplante Therapieereignis zu erinnern.

Die Vorrichtung 10 ist angepasst, es dem Nutzer zu ermöglichen, zu dem geplanten Therapieereignis eine Information zur Beschreibung des geplanten Therapieereignisses in dem Therapieplan bereitzustellen, und der elektronische Informationsträger 20 ist angepasst, die beschreibende Information für den Patienten wiederzugegeben, wenn der Patient an das geplante Therapieereignis erinnert wird. Die beschreibende Information kann eine durch den Nutzer frei editierbare Textinformation und/oder eine Audioinformation und/oder eine Standbildinformation und/oder eine Bewegtbildinformation umfassen.

In dieser Ausführungsform ist die Vorrichtung 10 zum Erstellen eines Therapieplans für einen Patienten durch einen Nutzer ein Computer, in diesem Beispiel ein Notebook, auf dem Programmcodemittel eines Computerprogramms ausgeführt werden, welche den Computer zu der Vorrichtung 10 weiterbilden.

Der elektronische Informationsträger 20 für den Patienten ist hier ein tragbares elektronisches Gerät, dessen Abmessungen bzw. dessen Gestalt im Wesentlichen denjenigen heutiger Smartphones entsprechen. Das Gerät umfasst, in dieser Ausführungsform, unter anderem ein Display 21, hier ein Touch-Screen-Display, zur Wiedergabe visueller Informationen und einen Lautsprecher 22 zur Wiedergabe akustischer Informationen. Die Displaygröße beträgt in diesem Beispiel um die 5 Zoll (zirka 12,7 cm) Displaydiagonale. Die Funktionalität des Erinnerns des Patienten an das geplante Therapieereignis ist durch eine Kombination eines visuellen und eines akustischen Alarms (hier ein Blinken und ein Piepen) realisiert. Der Alarm wird zu einem für das Therapieereignis in dem Therapieplan vorgesehenen Zeitpunkt ausgelöst.

In dieser Ausführungsform kann der durch den Nutzer für den Patienten erstellte Therapieplan mittels einer direkten drahtgebundenen Verbindung (in der Figur nicht gezeigt), insbesondere einem USB-Kabel, auf den elektronischen Informationsträger 20 für den Patienten übertragen und dort gespeichert werden. Zusätzlich ist in diesem Beispiel aber auch eine "indirekte" Übertragung des Therapieplans mittels eines portablen Speichermediums 1, insbesondere einem USB-Speicherstick, möglich.

Die Vorrichtung 10 umfasst hier eine Kamera 11 zur Aufnahme der Standbildinformation und ist angepasst, eine Bewegtbildinformation von der Kamera 11 als Vorschau für den Nutzer zu erhalten und wiederzugeben, und dem Nutzer zu ermöglichen, die Standbildinformation aus der als Vorschau erhaltenen und wiedergegebenen Bewegtbildinformation zu erfassen. Der Nutzer kann die Standbildinformation somit selbst erstellen. Dies ist in der Fig. 1 am Beispiel einer Standbildinformation, welche die Verpackung eines Medikamentes, einen Blister und eine entsprechende Tablette zeigen soll, dargestellt. Die Kamera 11, beispielsweise eine mit der Vorrichtung 10 über ein USB-Kabel verbundene Web-Kamera, erzeugt eine Bewegbildinformation, die von der Vorrichtung 10 erhalten und als Vorschau für den Nutzer z.B. in einem Vorschaufenster 12 wiedergegeben wird. Der Nutzer kann unter Zuhilfenahme der Vorschau-Bewegtbildinformation dann sehr einfach die Verpackung, den Blister und die Tablette so arrangieren, dass diese in einer von dem Nutzer gewünschten Anordnung oder Perspektive gezeigt werden. Sobald der Nutzer ein im passend erscheinendes Arrangement gefunden hat, kann er, z.B. durch Drücken eines "Capture"-Buttons des Computerprogramms, die Standbildinformation aus der als Vorschau erhaltenen und wiedergegebenen Bewegtbildinformation erfassen. Dabei wird das aktuelle Bild der Bewegtbildinformation automatisch als ein Standbild (z.B. als ein JPEGkomprimiertes Bild) in einem vorbestimmten Ordner abgespeichert.

Die Vorrichtung 10 ist, in dieser Ausführungsform, angepasst, einen in der Standbildinformation und/oder in der Bewegtbildinformation sichtbaren Code, insbesondere einen Strichcode und/oder einen Matrixcode, zu erkennen und zu interpretieren, und dem Nutzer zu ermöglichen, die beschreibende Information basierend auf dem erkannten und interpretierten Code bereitzustellen. Insbesondere kann die Vorrichtung 10, in diesem Beispiel, die auf der Verpackung eines Medikamentes aufgedruckte Klartext-Darstellung der Pharmazentralnummer (PZN) des Medikamentes bzw. die entsprechende Darstellung als Strichcode erkennen und interpretieren. Die in der Pharmazentralnummer codierten Informationen (z.B. Bezeichnung, Darreichungsform, Wirkstoffstärke und Verpackungsgröße eines Medikamentes) werden von der Vorrichtung dazu verwendet, dem Nutzer Vorschläge für die beschreibende Information zu unterbreiten. Dies geschieht hier dadurch, dass aus einer, z.B. in Form einer Datenbank, angelegten Sammlung von schon einmal in einem Therapieplan bereitgestellten beschreibenden Informationen (siehe unten), solche beschreibenden Informationen, die sich auf die Einnahme oder Anwendung des durch die erkannte PZN gekennzeichneten Medikamentes beziehen, ausgewählt und für den Nutzer in Form eines Auswahlmenüs, über das er die von ihm gewünschte beschreibende Information übernehmen kann, bereitgestellt werden.

Die Vorrichtung 10 ist hier ferner angepasst, eine Vorschau der Wiedergabe der beschreibenden Information, wie sie durch den elektronischen Informationsträger 20 für den Patienten erfolgen soll, für den Nutzer wiederzugeben. Dies erfolgt hier ebenfalls in einem entsprechenden Vorschaufenster (in der Figur nicht gezeigt) und ggf. über einen Lautsprecher (in der Figur ebenfalls nicht gezeigt). Der Nutzer kann so direkt sehen, wie die beschreibende Information später für den Patienten wiedergeben wird.

Darüberhinaus ist die Vorrichtung 10, in dieser Ausführungsform, angepasst, die durch den Nutzer in dem Therapieplan bereitgestellte beschreibende Information zur möglichen Wiederverwendung automatisch abzuspeichern. Auf diese Weise wird im Laufe der Zeit automatisch eine Sammlung von schon einmal in einem Therapieplan bereitgestellten beschreibenden Informationen, z.B. in Form einer Datenbank, angelegt, auf die der Nutzer bei der Erstellung eines Therapieplans für einen Patienten zurückgreifen kann.

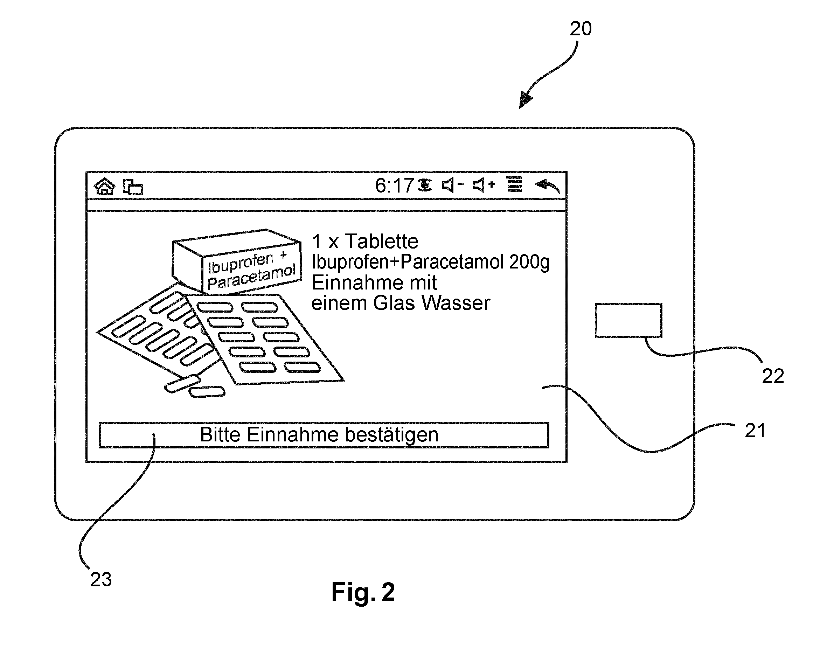

Fig. 2 zeigt schematisch und exemplarisch eine Detailansicht des elektronischen Informationsträgers 20 der in der Fig. 1 gezeigten Ausführungsform eines Systems 100 zur Förderung der Therapietreue von Patienten.

Der elektronische Informationsträger 20 ist in der Fig. 2 in einem Zustand gezeigt, in dem er einen Patienten basierend auf einem von einem Nutzer der in der Fig. 1 gezeigten Vorrichtung 10 an ein geplantes Therapieereignis erinnert. Dabei gibt er die beschreibende Information, d.h., die Information zur Beschreibung des geplanten Therapieereignisses, die der Nutzer zu dem geplanten Therapieereignis in dem Therapieplan bereitgestellt hat, für den Patienten wieder. In diesem Beispiel umfasst die beschreibende Information einen durch den Nutzer frei editierten Text, hier den Satz "Einnahme mit einem Glas Wasser" sowie eine Standbildinformation, welche die Verpackung des Medikamentes Ibuprofen + Paracetamol zeigt. Die weiteren Textelemente "1 x Tablette" und "Ibuprofen + Paracetamol 200mg" sind vordefinierte Texte, die in einer der Vorrichtung 10 zur Verfügung stehenden Datenbank hinterlegt sind und die dem Nutzer der Vorrichtung 10 beispielsweise in Form eines Listenauswahlmenüs zur Auswahl und zur Verwendung zur Verfügung gestellt werden. Dabei sieht die Vorrichtung 10 in dieser Ausführungsform auch die Möglichkeit vor, solche vordefinierten Texte individuell zu bearbeiten bzw. zu verändern, wobei die bearbeiteten bzw. veränderten Texte - wie oben beschrieben - zur möglichen Wiederverwendung automatisch abgespeichert werden.

Die beschreibende Information wird hier auf dem Touch-Screen-Display 21 des elektronischen Informationsträgers 20 visuell wiedergeben. Zusätzlich kann der elektronische Informationsträger 20 aber auch angepasst sein, eine Textinformation der beschreibenden Information, hier z.B. den Satz "Einnahme mit einem Glas Wasser", mittels eines Text-nach-Sprache-Wandlers akustisch über den Lautsprecher 22 wiederzugeben.

Der elektronische Informationsträger 20 ist hier ferner angepasst, von dem Patienten eine Bestätigung zu fordern, dass der Patient an das geplante Therapieereignis erinnert worden ist. Die Bestätigungsfunktionalität ist hier dadurch realisiert, dass auf dem Touch-Screen-Display 21 als Nutzerschnittstelle ein Bestätigungs-Button 23 wiedergegeben wird, welchen der Patient drücken muss, um den Alarm auszustellen. Auf die erfolgte Bestätigung durch den Patienten hin, wird ein Bestätigungsnachweis durch den elektronischen Informationsträger 20, z.B. in einem internen Speicher, gespeichert. In dieser Ausführungsform ist der elektronischen Informationsträger 20 angepasst, falls die beschreibende Information eine Audioinformation und/oder eine Bewegtbildinformation umfasst, diese solange wiederholt wiederzugeben, bis die Bestätigung durch den Patienten erfolgt ist.

Der elektronische Informationsträger 20 ist, in dieser Ausführungsform, angepasst, wenn das Therapieereignis die Einnahme oder Anwendung eines Medikamentes umfasst, basierend auf dem Bestätigungsnachweis eine Prognose über den dem Patienten verbleibenden Vorrat des Medikamentes aufzustellen und die Prognose visuell und akustisch wiederzugeben. Auf diese Weise wird der Patient über seinen jeweils aktuellen Medikamentenvorrat auf dem Laufenden gehalten werden, so dass er sich rechtzeitig um ein neues Medikament kümmern kann. In einer anderen Ausführungsform kann der elektronische Informationsträger 20 zusätzlich oder alternativ angepasst sein, die Prognose per Datenfernübertragung zu der Vorrichtung 10 zu übertragen. Dies kann dem Nutzer der Vorrichtung, z.B. einem Arzt oder Apotheker, helfen, die Lagersteuerung zu verbessern.

Der durch den Nutzer erstellte Therapieplan umfasst, je nach Gesundheitszustand des Patienten, ein oder mehrere geplante Therapieereignisse. In dieser Ausführungsform ist der elektronische Informationsträger 20 ferner angepasst, eine Gesamtübersicht über die ein oder mehreren geplanten Therapieereignisse visuell und/oder akustisch wiederzugeben. In der hier beschriebenen Realisierung weist der elektronische Informationsträger als Nutzerschnittstelle einen Informations-Button auf, der am unteren rechten Rand des Touch-Screen-Displays 21 vorgesehen ist (in der Figur nicht gezeigt). Wird dieser Informations-Button gedrückt, wird die Gesamtübersicht über die ein oder mehreren geplanten Therapieereignisse als Übersichtsliste auf dem Touch-Screen-Display 21 wiedergegeben. Zusätzlich wird eine Information über die "Heimat"-Apotheke des Patienten, hier die komplette Adresse der Apotheke, auf dem Touch-Screen-Display 21 wiedergegeben. (Die Wiedergabe der Übersichtsliste über die ein oder mehreren geplanten Therapieereignisse sowie die Wiedergabe der Adresse der "Heimat"-Apotheke des Patienten sind in der Figur nicht gezeigt.)

In dieser Ausführungsform ist das System angepasst, eine unbefugte Wiederverwendung zumindest eines Teils der in dem Therapieplan bereitgestellten beschreibenden Information zu verhindern. Insbesondere ist die beschreibende Information, soweit es sich um eine von dem Nutzer selbst erstellte beschreibende Information handelt, verschlüsselt. In einer anderen Ausführungsform kann das System zusätzlich oder alternativ aber auch angepasst sein, die Wiederwendung der in dem Therapieplan bereitgestellten beschreibenden Information nach einer Wiedergabe einer Erklärung über mögliche bestehende Rechte an zumindest einem Teil der in dem Therapieplan bereitgestellten beschreibenden Information zu ermöglichen. Dabei kann die genannte Erklärung einen Hinweis auf den möglichen Rechteinhaber beinhalten.

Weitere Variationen der offenbarten Ausführungsformen können von einem die beanspruchte Erfindung praktizierenden Fachmann aus einer Betrachtung der Zeichnungen, der Beschreibung und der beigefügten Ansprüche verstanden und ausgeführt werden.

In den Ansprüchen schließen die Wörter "aufweisen" und "umfassen" nicht andere Elemente oder Schritte aus und der unbestimmte Artikel "ein" schließt eine Mehrzahl nicht aus.

Eine einzelne Einheit oder Vorrichtung kann die Funktionen mehrerer Elemente durchführen, die in den Ansprüchen aufgeführt sind. Die Tatsache, dass einzelne Funktionen und/oder Elemente in unterschiedlichen abhängigen Ansprüchen aufgeführt sind, bedeutet nicht, dass nicht auch eine Kombination dieser Funktionen und/oder Elemente vorteilhaft verwendet werden könnte.

Ein Computerprogramm kann auf einem geeigneten Medium gespeichert und/oder verteilt werden, wie beispielsweise einem optischen Speichermedium oder einem Festkörperspeichermedium, das zusammen mit oder als Teil anderer Hardware vertrieben wird. Das Computerprogramm kann aber auch in anderen Formen vertrieben werden, beispielsweise über das Internet oder andere Telekommunikationssysteme.

Die Bezugszeichen in den Ansprüchen sind nicht derart zu verstehen, dass der Gegenstand und der Schutzbereich der Ansprüche durch diese Bezugszeichen eingeschränkt wäre.

## Patentansprüche

1. System (100) zur Förderung der Therapietreue von Patienten, wobei das System (100) umfasst:
- eine Vorrichtung (10) zum Erstellen eines Therapieplans für einen Patienten durch einen Nutzer, insbesondere durch einen Heilkundigen, wobei der Therapieplan ein geplantes Therapieereignis umfasst, und
- einen elektronischen Informationsträger (20) für den Patienten, welcher angepasst ist, den Patienten basierend auf dem Therapieplan an das geplante Therapieereignis zu erinnern,
wobei die Vorrichtung (10) angepasst ist, dem Nutzer zu ermöglichen, zu dem geplanten Therapieereignis eine Information zur Beschreibung des geplanten Therapieereignisses in dem Therapieplan bereitzustellen,
wobei der elektronische Informationsträger (20) angepasst ist, die beschreibende Information für den Patienten wiederzugeben, wenn der Patient an das geplante Therapieereignis erinnert wird, und
wobei die beschreibende Information eine durch den Nutzer frei editierbare Textinformation und/oder eine Audioinformation und/oder eine Standbildinformation und/oder eine Bewegtbildinformation umfassen kann.

2. System (100) nach Anspruch 1, wobei die Vorrichtung (10) eine Kamera (11) zur Aufnahme der Standbildinformation umfasst und angepasst ist, eine Bewegtbildinformation von der Kamera (11) als Vorschau für den Nutzer zu erhalten und wiederzugeben, und dem Nutzer zu ermöglichen, die Standbildinformation aus der als Vorschau erhaltenen und wiedergegebenen Bewegtbildinformation zu erfassen.

3. System (100) nach Anspruch 1 oder 2, wobei die Vorrichtung (10) angepasst ist, einen in der Standbildinformation und/oder in der Bewegtbildinformation sichtbaren Code, insbesondere einen Strichcode und/oder einen Matrixcode, zu erkennen und zu interpretieren, und dem Nutzer zu ermöglichen, die beschreibende Information basierend auf dem erkannten und interpretierten Code bereitzustellen.

4. System (100) nach einem der Ansprüche 1 bis 3, wobei die Vorrichtung (10) angepasst ist, eine Vorschau der Wiedergabe der beschreibenden Information, wie sie durch den elektronischen Informationsträger (20) für den Patienten erfolgen soll, für den Nutzer wiederzugeben.

5. System (100) nach einem der Ansprüche 1 bis 4, wobei die Vorrichtung (10) angepasst ist, die durch den Nutzer in dem Therapieplan bereitgestellte beschreibende Information zur möglichen Wiederverwendung automatisch abzuspeichern.

6. System (100) nach einem der Ansprüche 1 bis 5, wobei der elektronische Informationsträger (20) angepasst ist, eine Textinformation der beschreibenden Information mittels eines Text-nach-Sprache-Wandlers akustisch wiederzugeben.

7. System (100) nach einem der Ansprüche 1 bis 6, wobei der elektronische Informationsträger (20) angepasst ist, von dem Patienten eine Bestätigung zu fordern, dass der Patient an das geplante Therapieereignis erinnert worden ist, und
- einen Bestätigungsnachweis für die durch den Patienten erfolgte Bestätigung zu speichern und/oder per Datenfernübertragung zu der Vorrichtung (10) zu übertragen, und/oder
- die Audioinformation und/oder die Bewegtbildinformation solange wiederholt wiederzugeben, bis die Bestätigung durch den Patienten erfolgt ist.

8. System nach Anspruch 7, wobei das Therapieereignis die Einnahme oder Anwendung eines Medikamentes umfasst, wobei der elektronische Informationsträger (20) angepasst ist, basierend auf dem Bestätigungsnachweis eine Prognose über den dem Patienten verbleibenden Vorrat des Medikamentes aufzustellen, wobei der elektronische Informationsträger (20) des Weiteren angepasst ist, die Prognose visuell und/oder akustisch wiederzugeben und/oder per Datenfernübertragung zu der Vorrichtung (10) zu übertragen.

9. System (100) nach einem der Ansprüche 1 bis 8, wobei der Therapieplan ein oder mehrere geplante Therapieereignisse umfasst, wobei der elektronische Informationsträger (20) angepasst ist, eine Gesamtübersicht über die ein oder mehreren geplanten Therapieereignisse visuell und/oder akustisch wiederzugeben.

10. System (100) nach einem der Ansprüche 1 bis 9, wobei der elektronische Informationsträger (20) ein tragbares elektronisches Gerät ist, bei dem die Funktionalität des Erinnerns des Patienten an das geplante Therapieereignis und der Wiedergabe der beschreibenden Information für den Patienten gegenüber möglichen weiteren Funktionen im Vordergrund steht.

11. System nach einem der Ansprüche 1 bis 10, wobei die System (100) angepasst ist
- eine unbefugte Wiederverwendung zumindest eines Teils der in dem Therapieplan bereitgestellten beschreibenden Information zu verhindern, und/oder
- die Wiederwendung der in dem Therapieplan bereitgestellten beschreibenden Information nach einer Wiedergabe einer Erklärung über mögliche bestehende Rechte an zumindest einem Teil der in dem Therapieplan bereitgestellten beschreibenden Information zu ermöglichen.

12. Vorrichtung (10) zum Erstellen eines Therapieplans für einen Patienten durch einen Nutzer, insbesondere durch einen Heilkundigen, zur Verwendung in dem System (100) zur Förderung der Therapietreue von Patienten gemäß einem der Ansprüche 1 bis 11.

13. Elektronischer Informationsträger (20) für einen Patienten zur Verwendung in dem System (100) zur Förderung der Therapietreue von Patienten gemäß einem der Ansprüche 1 bis 11.

14. Computerprogramm, wobei das Computerprogramm Programmcodemittel umfasst, welche, wenn sie auf einem Computer ausgeführt werden, den Computer zu einer Vorrichtung (10) zum Erstellen eines Therapieplans für einen Patienten durch einen Nutzer, insbesondere durch einen Heilkundigen, zur Verwendung in dem System (100) zur Förderung der Therapietreue von Patienten gemäß einem der Ansprüche 1 bis 11 weiterbilden.

15. Computerprogramm, wobei das Computerprogramm Programmcodemittel umfasst, welche, wenn sie auf einem tragbaren elektronischen Gerät mit Computerfunktionalität ausgeführt werden, das tragbare elektronische Gerät mit Computerfunktionalität zu einem elektronischen Informationsträger (10) ) für einen Patienten zur Verwendung in dem System (100) zur Förderung der Therapietreue von Patienten gemäß einem der Ansprüche 1 bis 11 weiterbilden.
